# EUROPÄISCHE PATENTSCHRIFT

(11) **EP 2 338 435 B1**
(45) Veröffentlichungstag und Bekanntmachung des Hinweises auf die Patenterteilung: **28.10.2015**
(21) Anmeldenummer: 10015163.8
(22) Anmeldetag: 01.12.2010
(51) Int. Cl.: A61B 19/00, F16M 11/14, A61B 17/00

(54) **Haltevorrichtung für medizinische Instrumente**
Holder for medical instruments
Dispositif de retenue pour instruments médicaux

(30) Priorität: 23.12.2009 DE 102009060494
(43) Veröffentlichungstag der Anmeldung: 29.06.2011
(73) Patentinhaber: Karl Storz GmbH & Co. KG, 78532 Tuttlingen (DE)
(72) Erfinder: Stefan, Jochen, 78532 Tuttlingen (DE); Bacher, Uwe, 78532 Tuttlingen (DE)

(56) Entgegenhaltungen:
- EP-A1- 0 868 885
- EP-A2- 0 198 933
- WO-A1-91/05960
- DE-A1- 19 521 060
- US-A- 2 533 494
- US-B1- 6 767 153

## Beschreibung

Die Erfindung betrifft eine Haltevorrichtung für medizinische Instrumente, mit einem Tragarm, an dem mindestens ein medizinisches Instrument festlegbar ist und mit mindestens einem Gelenk zur Positionierung des Tragarms und/oder des medizinischen Instruments, wobei das mindestens eine Gelenk als mit mindestens einer Lagerschale und einer Gelenkkugel versehenes Kugelgelenk ausgebildet ist, das zwischen einem das Gelenk freigebenden und einem das Gelenk arretierenden Zustand verstellbar ist, wobei die Lagerschale starr ausgebildet ist und die Gelenkkugel über mehrere Sperrelemente klemmend in der Lagerschale festlegbar ist und wobei die Lagerschale aus einer oberen Lagerschale und einer unteren Lagerschale bestehend zweiteilig ausgebildet ist und mehrere Ausnehmung für die Sperrelemente in der unteren Lagerschale ausgebildet sind.

Derartige Haltevorrichtungen sind bei der Durchführung chirurgischer Eingriffe häufig erforderlich, um medizinische Instrumente verschiedenster Art, wie beispielsweise Retraktoren, Kameras oder Endoskope, über einen längeren Zeitraum in einer bestimmten Position zu halten. Durch die gelenkige Ausgestaltung der Haltevorrichtungen ist es für den Chirurgen möglich, das von dem Tragarm gehaltene medizinische Instrument exakt zu positionieren und die eingestellte Position der Haltevorrichtung durch Arretieren des Gelenks bzw. der Gelenke des Tragarms zu fixieren.

Eine gattungsgemäße Haltevorrichtung ist aus der US 2 533 494 A bekannt. Diese Druckschrift offenbart eine einstellbare Stütze mit einem Tragarm mit mindestens einem als Kugelgelenk ausgebildeten Gelenk zur Positionierung der Stütze. Das Arretieren der Gelenkkugel erfolgt über als elektrisch betätigbare Kolben ausgebildete Sperrelemente, die mit angeformten Verriegelungszapfen in Rastmulden eingreifen, die in der Oberfläche der Gelenkkugel ausgebildet sind.

Nachteilig an dieser bekannten Arretierung der Gelenkkugel ist einerseits, dass die Gelenkkugel nur in vorgegebenen Rastpositionen, nämlich den Rastmulden in der Gelenkkugel positionierbar ist und andererseits, dass bei einem Stromausfall oder einem versehentlichen Abschalten der Stromzufuhr zu den Kolben die Arretierung der Gelenkkugel und somit der gesamtem Stütze aufgehoben wird.

Eine Haltevorrichtung ist beispielsweise aus der DE 295 21 305 U1 bekannt. Bei dieser bekannten Haltevorrichtung wird die Gelenkkugel über eine federbelastete Andrückplatte gegen die Lagerschale gedrückt, die ihrerseits einen Teil der Lagerschale bildet. Zum Arretieren des Gelenks ist die Andrückplatte mit einem piezo-elektrischen Aktor gekoppelt ist, der sich beim Anlegen einer elektrischen Spannung ausdehnt und so den Druck der Andrückplatte auf die Gelenkkugel erhöht, bis diese klemmend fixiert wird.

Mit dieser bekannten Konstruktion ist es zwar möglich, das Kugelgelenk sicher zu fixieren, jedoch weist die Konstruktion den Nachteil auf, dass bei Stromausfall keine Arretierung des Gelenks mehr möglich ist. Darüber hinaus weisen Konstruktionen mit einer teilweise federbelasteten Lagerschale den Nachteil auf, dass sich das Gelenk bei einer Axialkraft auf die Gelenkkugel, die entgegen der Kraftrichtung der federbelasteten Lagerschale gerichtet ist, ungewollt lösen kann.

Weitere Haltearme sind aus der WO 91/05960 A1, EP 0 198 933 A2 und der DE 195 21 060 A1 bekannt, die sich nicht durch eine sichere einfache Handhabung auszeichnen.

In der EP 0 868 885 A1 ist ein Haltearm für eine Lagerschale für den Arm eines Chirurgen offenbart, die mit einem Gelenk versehen ist, das eine Gelenkkugel und eine diese umfassende Lagerschale aufweist. Das Gelenk kann mithilfe eines Verschlussstößels so in einen arretierten Zustand versetzt werden, dass der Stößel die Gelenkkugel so in den Lagersitz flächig hinein presst, dass das Gelenk arretiert ist. Wird der Stößel von der Gelenkkugel abgehoben, ist dieses Gelenk frei beweglich. Dieser Haltearm erweist sich als wenig sicher im Hinblick einfache Handhabung und schnelle Fixierung.

Davon ausgehend liegt der Erfindung die Aufgabe zugrunde, eine Haltevorrichtung für medizinische Instrumente der eingangs genannten Art zu schaffen, die bei einfacher Handhabung ein schnelles Arretieren und Lösen des mindestens einen Gelenks ermöglicht und darüber hinaus sicherstellt, dass ein unbeabsichtigtes Lösen der Arretierung weitestgehend ausgeschlossen ist.

Die Lösung dieser Aufgabenstellung ist erfindungsgemäß durch die Merkmale des Anspruchs 1 gegeben. Dabei ist liegen die Sperrelemente im das Gelenk arretierenden Zustand flächig an der Gelenkkugel an und drücken die Gelenkkugel klemmend in die Lagerschale, wobei jedes Sperrelement über ein Federelement in Richtung auf die Anlage an der Gelenkkugel vorgespannt ist und alle Sperrelemente über ein gemeinsames Betätigungselement in eine das Gelenk freigebende Stellung überführbar sind.

Durch die erfindungsgemäße Ausbildung der Sperrelemente, über die die Gelenkkugel in der starren, also in ihrer Ausbildung weitgehend unveränderbaren Lagerschale klemmend gehalten wird, wird sichergestellt, dass auch bei einer auf die Gelenkkugel ausgeübten Axialkraft die die Gelenkkugel umschließende Lagerschale unverändert bleibt und das mindestens eine Sperrelement die Gelenkkugel klemmend arretieren kann.

In der Lagerschale sind Ausnehmung ausgebildet, über die jedes Sperrelement so in das Gelenk einführbar ist, dass das Sperrelement die Gelenkkugel zum Arretieren des Gelenks klemmend gegen die Lagerschale drückt. Die über Ausnehmungen in der Lagerschale einführbaren Sperrelemente ermöglichten die erfindungsgemäße Arretierung der Gelenkkugel ohne die Lagerschale zu Klemmzwecken in ihrer Lage oder Dimensionierung verändern zu müssen.

Die Ausgestaltungsform der Lagerschale aus einer oberen Lagerschale und einer unteren Lagerschale bestehend stellt eine in der Herstellung und Montage besonders leicht zu handhabende Konstruktion zur Ausbildung der Lagerschale dar.

Jedes Sperrelement ist über ein Federelement in Richtung auf die Anlage an der Gelenkkugel vorgespannt, um eine dauerhafte Anlage der Sperrelemente an der Gelenkkugel zu gewährleisten und ein unbeabsichtigtes Lösen der Arretierung weitestgehend auszuschließen. Zum Lösen der arretierenden Klemmung der Gelenkkugel sind alle Sperrelemente über ein gemeinsames Betätigungselement in eine das Gelenk freigebende Stellung überführbar sind, um ein schnelles und gleichmäßiges Lösen der Arretierung zu gewährleisten. Das Betätigungselement ist so ausgebildet, dass es jedes Sperrelement aus der Anlage an der Gelenkkugel zurückzieht.

Um eine gleichmäßige Anlage der Sperrelemente an der Gelenkkugel und somit eine gleichmäßige Klemmung und Fixierung der Gelenkkugel zu erzielen wird erfindungsgemäß vorgeschlagen, dass über den Umfang der Lagerschale verteilt vier Ausnehmungen für jeweils mindestens ein Sperrelement in der Lagerschale ausgebildet sind.

Um das klemmende Eindrücken der Gelenkkugel in die Lagerschale mittels der Sperrelemente zu erleichtern, wird mit der Erfindung weiterhin vorgeschlagen, dass jedes Sperrelement zumindest im Bereich der Anlage an der Gelenkkugel konisch ausgebildet ist, wobei vorteilhafterweise an jedem Sperrelement im Bereich der Anlage des Sperrelements an der Gelenkkugel ein Klemmbelag angeordnet sein kann. Als Klemmbelag kann beispielsweise ein Material mit einem hohen Reibwiderstand verwendet werden, um eine Relativbewegung zwischen dem Sperrelement und der Gelenkkugel zu verhindern.

Weiterhin wird mit der Erfindung vorgeschlagen, dass das Betätigungselement durch das Anlegen einer elektrischen Spannung betätigbar ist, wobei das Betätigungselement vorteilhafterweise über mindestens einen piezo-elektrischen Aktor betätigbar ist. Piezo-elektrische Aktoren zeichnen sich dadurch aus, dass sie beim Anlegen einer elektrischen Spannung mit einer Längenänderung, beispielsweise einem Ausdehnen, reagieren. Durch das Ausdehnen des mit elektrischer Spannung beaufschlagten piezo-elektrischen Aktors werden die Sperrelemente über das Betätigungselement von der Gelenkkugel fortgezogen und so das Gelenk wieder freigegeben.

Schließlich wird mit der Erfindung vorgeschlagen, dass das Kugelgelenk durch mindestens ein an der Gelenkkugel anliegendes Dichtungselement gegenüber der Umgebung abgedichtet ist, um ein gegen äußere Einflüsse, wie insbesondere Feuchtigkeit und Schmutz, geschütztes Gelenk zu erhalten.

Weitere Merkmale und Vorteile der Erfindung ergeben sich anhand der zugehörigen Zeichnungen, in denen ein Ausführungsbeispiel einer erfindungsgemäßen Haltevorrichtung für medizinische Instrumente nur beispielhaft dargestellt ist, ohne die Erfindung auf dieses Ausführungsbeispiel zu beschränken. In den Zeichnungen zeigt:
- Fig. 1: eine Haltevorrichtung für medizinische Instrumente gemäß dem Stand der Technik;
- Fig. 2: eine Schnittdarstellung eines Kugelgelenks für eine erfindungsgemäße Haltevorrichtung für medizinische Instrumente und
- Fig. 3: eine perspektivische Ansicht des Kugelgelenks gemäß Fig.2.

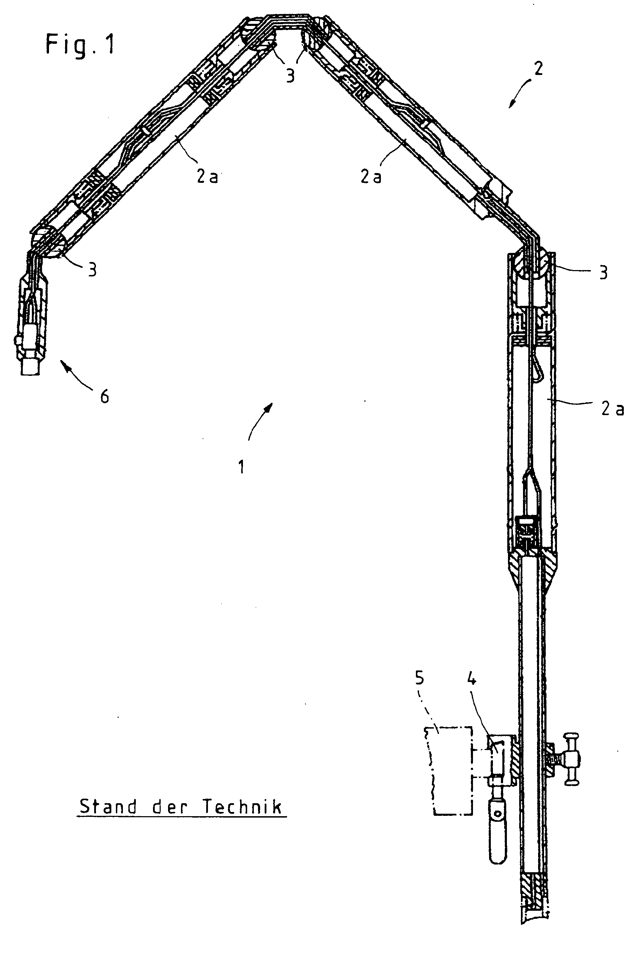

Die Abbildung Fig. 1 zeigt eine Haltevorrichtung 1 für medizinische Instrumente gemäß dem Stand der Technik.

Diese Haltevorrichtung 1 besteht im Wesentlichen aus einem aus mehreren Tragarmteilen 2a bestehenden Tragarm 2, wobei die einzelnen Tragarmteile 2a des Tragarms über als Kugelgelenke 3 ausgebildete Gelenke 3 relativ zueinander verschwenkbar miteinander verbunden sind.

Derartige Haltevorrichtungen 1 sind bei der Durchführung chirurgischer Eingriffe häufig erforderlich, um medizinische Instrumente verschiedenster Art, wie beispielsweise Retraktoren, Kameras oder Endoskope, über einen längeren Zeitraum in einer bestimmten Position zu halten. Durch die gelenkige Ausgestaltung der Haltevorrichtung 1 ist es für den Chirurgen möglich, das medizinische Instrument exakt zu positionieren und die eingestellte Position der Haltevorrichtung 1 durch Arretieren des Gelenks 3 bzw. der Gelenke 3 zu fixieren. Neben der endoskopischen Chirurgie finden derartige Haltevorrichtungen 1 auch in der offenen Chirurgie Anwendung.

Im Bereich seines proximalen Endes ist der Tragarm 2 über eine Einspannvorrichtung 4, beispielsweise am Operationstisch 5, festlegbar. Am distalen Ende weist der Tragarm 2 eine Instrumentenaufnahme 6 zur Aufnahme des über die Haltevorrichtung 1 zu positionierenden medizinischen Instruments auf.

Alternativ zu dem in Fig. 1 dargestellten Aufbau des Tragarms 2 aus mehreren hintereinander gekoppelten Tragarmteilen 2a, die über Gelenke 3 miteinander verbunden sind, ist es selbstverständlich auch möglich den Tragarm 2 mehrarmig so auszugestalten, dass ausgehend von einem Gelenk 3 mehrere Tragarmteile 2a in verschiedene Richtungen zeigen. Diese Tragarmteile 2a ihrerseits können wieder über Gelenke 3 mit weiteren Tragarmteilen 2a gekoppelt sein und an ihren distalen Enden jeweils mit Instrumentenaufnahmen 6 zur Aufnahme der über die Haltevorrichtung 1 zu positionierenden medizinischen Instrumente ausgestattet sein.

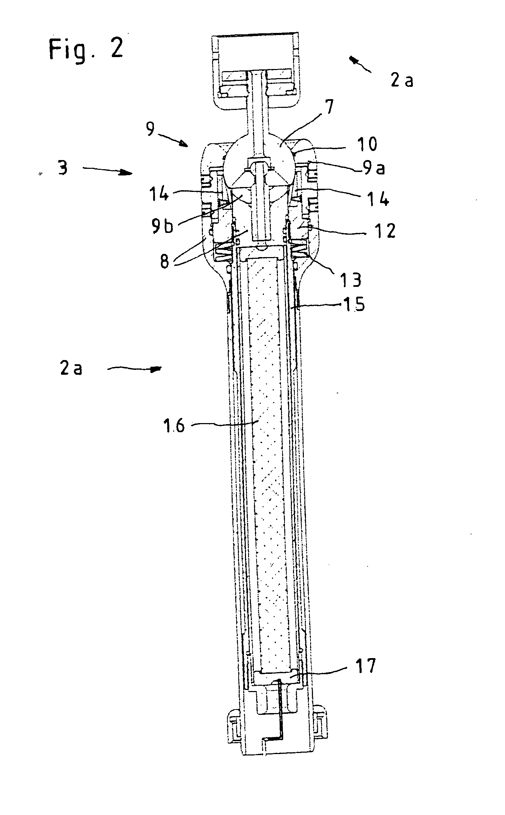

Der Aufbau der Kugelgelenke 3 ist den Abbildungen Fig. 2.und 3 zu entnehmen.

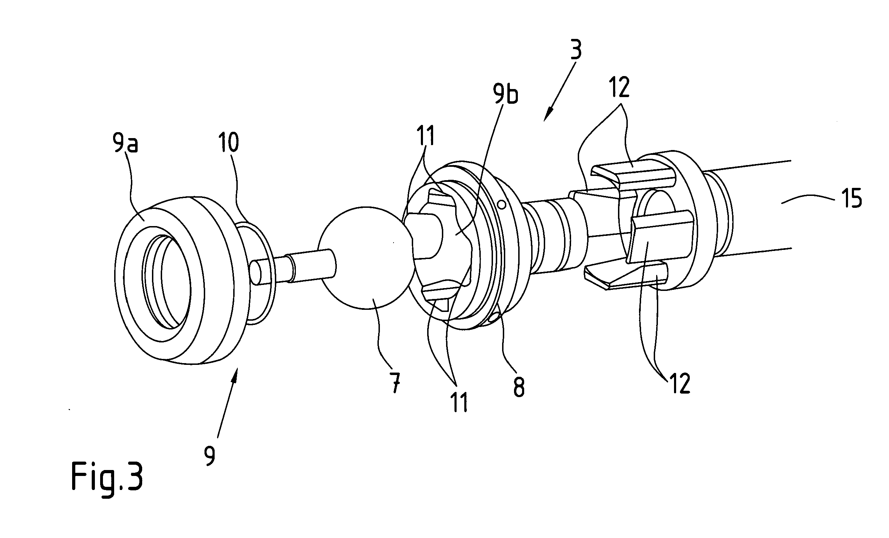

Wie aus Fig. 2 und 3 ersichtlich, bestehen die Kugelgelenke 3 aus einer Gelenkkugel 7, die verschwenkbar in einer in einem Gelenkgehäuse 8 ausgebildeten Lagerschale 9 gelagert ist. Bei dem dargestellten Ausführungsbeispiel ist die Lagerschale 9 aus einer oberen Lagerschale 9a und einer unteren Lagerschale 9b bestehend zweiteilig ausgebildet. Die Lagerschale 9 ist starr im Gelenkgehäuse 8 angeordnet, das heißt, dass die obere Lagerschale 9a und die untere Lagerschale 9b im montierten Zustand des Kugelgelenks 3 in Axialrichtung des Gelenks 3 nicht relativ zueinander bewegbar sind, also keine Vergrößerung oder Verringerung des Bewegungsspiels der Gelenkkugel 7 in der Lagerschale 9 aufgrund einer Bewegung der Lagerschale 9 möglich ist.

Wie weiterhin aus Fig. 2 und 3 ersichtlich, weist das Kugelgelenk 3 in der oberen Lagerschale 9a ein an der Gelenkkugel 7 anliegendes Dichtungselement 10 auf, um das Gelenk 3 nach außen und somit gegenüber äußeren Einflüssen, wie beispielsweise Feuchtigkeit und Schmutz, abzukapseln.

Um das Kugelgelenk 3 in der jeweils erforderlichen Position des Tragarms 2 fixieren zu können, sind in der unteren Lagerschale 9b Ausnehmungen 11 ausgebildet, über die Sperrelemente 12 so in das Kugelgelenk 3 einführbar sind, dass die Sperrelemente 12 an der Gelenkkugel 7 anliegen und die Gelenkkugel klemmend in die Lagerschale 9 drücken. Wie aus einer Zusammenschau der Abbildungen Fig. 2 und 3 ersichtlich, sind die Sperrelemente 12 bei der dargestellten Ausführungsform so angeordnet, dass sie in etwa tangential an der Gelenkkugel 7 angreifen.

Alternativ zu der dargestellten Ausgestaltungsform ist es selbstverständlich auch möglich die Sperrelemente 12 so anzuordnen, dass sie überwiegend radial auf die Gelenkkugel 7 einwirken.

Die Klemmkraft zum Fixieren der Gelenkkugel 7 wird über Federelemente 13 aufgebracht, über die jedes Sperrelement 12 in Richtung auf die Anlage an der Gelenkkugel 7 vorgespannt ist.

Um das klemmende Eindrücken der Gelenkkugel 7 in die Lagerschale 9 mittels der Sperrelemente 12 zu erleichtern, ist jedes Sperrelement 12 zumindest im Bereich der Anlage an der Gelenkkugel 7 konisch ausgebildet ist, wobei bei dem dargestellten Ausführungsbeispiel an jedem Sperrelement 12 im Bereich der Anlage des Sperrelements 12 an der Gelenkkugel 7 ein Klemmbelag 14 angeordnet ist. Als Klemmbelag 13 kann beispielsweise ein Material mit einem hohen Reibwiderstand verwendet werden, um eine Relativbewegung zwischen dem Sperrelement 12 und der Gelenkkugel 7 zu verhindern.

Die Ausbildung des Kugelgelenks 3 mit den in das Innere des Gelenks 3 einführbaren federbelasteten Sperrelementen 12 zeigt, dass die Sperrelemente 12 auch beim Aufbringen äußerer Axialkräfte auf die Gelenkkugel 7, die entgegen der Druckrichtung der Federelemente 13 wirken, dauerhaft klemmend an der Gelenkkugel 7 anliegen, da die Lagerschale 9 starr im Gelenkgehäuse 8 angeordnet ist und so die aufgebrachten Axialkräfte ohne eine eigene axiale Verlagerung aufnimmt.

Das Lösen der Sperrelemente 12 und somit auch das Lösen der Arretierung des Gelenks 3 erfolgt über ein an den Sperrelementen 12 angreifendes Betätigungselement 15, das bei der in Fig. 2 dargestellten Ausführungsform als innerhalb des Gelenkgehäuses 8 gelagertes und an allen Sperrelementen 12 angreifendes Rohr ausgebildet ist. Über das Betätigungselement 15 können die Sperrelemente 12 gegen die Federkraft der Federelemente 13 aus ihrer Anlage an der Gelenkkugel 7 zurückgezogen werden, so dass die Gelenkkugel 7 wieder frei verschwenkbar in der Lagerschale 9 gelagert ist.

Bei der dargestellten Ausführungsform werden alle Sperrelemente 12 über ein gemeinsames Betätigungselement 15 in die das Gelenk 3 freigebende Position überführt. Selbstverständlich ist es auch möglich, pro Sperrelement 12 ein Betätigungselement 15 vorzusehen.

Die Aktivierung des Betätigungselements 15 erfolgt bei der dargestellten Ausführungsform durch das Anlegen einer elektrischen Spannung. Hierzu ist das Betätigungselement 15 mit einem piezo-elektrischen Aktor 16 gekoppelt, der sich beim Anlegen einer elektrischen Spannung ausdehnt.

Bei der in Fig. 2 dargestellten Ausführungsform stützt sich der piezo-elektrische Aktor 16 an einem inneren zentralen Teil des festen Gelenkgehäuses 8 ab. Sobald der Operateur zum Verstellen der Haltevorrichtung 1 den piezo-elektrischen Aktor 16, beispielsweise über einen Fußschalter, mit Spannung beaufschlagt, dehnt sich dieser aufgrund des piezoelektrischen Effekts aus, das heißt auf der Abbildung Fig. 2 nach unten und fort vom Kugelgelenk 3. Hierdurch wird das Betätigungselement 15, das über ein Mitnahmeelement 17, an dem der piezo-elektrischen Aktors 16 auf der dem Gelenk 3 abgewandten Seite anliegt, ebenfalls fort vom Kugelgelenk 3 gedrückt. Dies wiederum bewirkt, dass die direkt mit dem Betätigungselement 15 gekoppelten Sperrelemente 12 entgegen der Druckkraft der Federelemente 13 aus ihrer Anlage an der Gelenkkugel 7 gezogen werden und das Gelenk 3 freigegeben.

Sobald das Betätigungselement 15 nicht mehr betätigt wird bzw. der piezo-elektrische Aktor 16 nicht mehr mit elektrischer Spannung beaufschlagt wird, drücken die Federelemente 13 die Sperrelemente 12 zurück in die klemmende Anlage an der Gelenkkugel 7, wodurch das Gelenk 3 wieder arretiert wird. Somit handelt es sich bei dieser Ausführung um eine echte und wirkungsvolle Fail-Safe-Schaltung, da beispielsweise bei einem Stromausfall oder einem anderen Fehler des Betätigungselements 15 die Sperrelemente 12 immer in ihrer klemmenden und das Gelenk 3 arretierenden Anlage an der Gelenkkugel 7 verbleiben, bzw. wieder diese das Gelenk 3 sichernde Position einnehmen.

Eine wie voranstehend beschrieben ausgebildete Haltevorrichtung für medizinische Instrumente zeichnet sich dadurch aus, dass bei einfacher Handhabung ein schnelles Arretieren und Lösen des mindestens einen Gelenks 3 möglich ist und darüber sicherstellt ist, dass ein unbeabsichtigtes Lösen der Arretierung des Gelenks 3 weitestgehend ausgeschlossen ist.

**Bezugszeichenliste**

| | | | |
|---|---|---|---|
| 1 | Haltevorrichtung | 15 | Betätigungselement |
| 2 | Tragarm | 16 | Aktor |
| 2a | Tragarmteil | 17 | Mitnahmeelement |
| 3 | Gelenk / Kugelgelenk | | |
| 4 | Einspannvorrichtung | | |
| 5 | Operationstisch | | |
| 6 | Instrumentenaufnahme | | |
| 7 | Gelenkkugel | | |
| 8 | Gelenkgehäuse | | |
| 9 | Lagerschale | | |
| 9a | obere Lagerschale | | |
| 9b | untere Lagerschale | | |
| 10 | Dichtungselement | | |
| 11 | Ausnehmung | | |
| 12 | Sperrelement | | |
| 13 | Federelement | | |
| 14 | Klemmbelag | | |

## Patentansprüche

1. Haltevorrichtung für medizinische Instrumente, mit einem Tragarm (2), an dem mindestens ein medizinisches Instrument festlegbar ist und mit mindestens einem Gelenk (3) zur Positionierung des Tragarms (2) und/oder des medizinischen Instruments, wobei das mindestens eine Gelenk (3) als mit mindestens einer Lagerschale (9) und einer Gelenkkugel (7) versehenes Kugelgelenk (3) ausgebildet ist, das zwischen einem das Gelenk (3) freigebenden und einem das Gelenk (3) arretierenden Zustand verstellbar ist, wobei die Lagerschale (9) starr ausgebildet ist und die Gelenkkugel (7) über mehrere Sperrelemente (12) klemmend in der Lagerschale (9) festlegbar ist und wobei die Lagerschale (9) aus einer oberen Lagerschale (9a) und einer unteren Lagerschale (9b) bestehend zweiteilig ausgebildet ist und mehrere Ausnehmung (11) für die Sperrelemente (12) in der unteren Lagerschale (9b) ausgebildet sind,
**dadurch gekennzeichnet,**
**dass** die Sperrelemente (12) im das Gelenk (3) arretierenden Zustand flächig an der Gelenkkugel (7) anliegen und die Gelenkkugel (7) klemmend in die Lagerschale (9) drücken und, dass jedes Sperrelement (12) über ein Federelement (13) in Richtung auf die Anlage an der Gelenkkugel (7) vorgespannt ist und alle Sperrelemente (12) über ein gemeinsames Betätigungselement (15) in eine das Gelenk (3) freigebende Stellung überführbar sind.

2. Haltevorrichtung nach Anspruch 1, **dadurch gekennzeichnet, dass** über den Umfang der Lagerschale (9) verteilt vier Ausnehmungen (11) für jeweils mindestens ein Sperrelement (12) in der Lagerschale (9) ausgebildet sind.

3. Haltevorrichtung nach Anspruch 1 oder 2, **dadurch gekennzeichnet, dass** an jedem Sperrelement (12) im Bereich der Anlage des Sperrelements (12) an der Gelenkkugel (7) ein Klemmbelag (14) angeordnet ist.

4. Haltevorrichtung nach einem der Ansprüche 1 bis 3, **dadurch gekennzeichnet, dass** jedes Sperrelement (12) zumindest im Bereich der Anlage an der Gelenkkugel (7) konisch ausgebildet ist.

5. Haltevorrichtung nach einem der Ansprüche 1 bis 4 **dadurch gekennzeichnet, dass** das gemeinsame Betätigungselement (15) so ausgebildet ist, dass es jedes Sperrelement (12) aus der Anlage an der Gelenkkugel (7) zurückzieht.

6. Haltevorrichtung nach einem der Ansprüche 1 bis 5, **dadurch gekennzeichnet, dass** das Betätigungselement (15) durch das Anlegen einer elektrischen Spannung betätigbar ist.

7. Haltevorrichtung nach einem der Ansprüche 1 bis 6, **dadurch gekennzeichnet, dass** das mindestens eine Betätigungselement (15) über mindestens einen piezo-elektrischen Aktor (16) betätigbar ist.

8. Haltevorrichtung nach einem der Ansprüche 1 bis 7, **dadurch gekennzeichnet, dass** das Kugelgelenk (3) durch mindestens ein an der Gelenkkugel (7) anliegendes Dichtungselement (10) gegenüber der Umgebung abgedichtet ist.

## Claims

1. Holding device for medical instruments, with a bracket (2) on which at least one medical instrument can be secured, and with at least one joint (3) for positioning the bracket (2) and/or the medical instrument, wherein the at least one joint (3) is designed as a ball-and-socket joint (3) which is provided with at least one bearing shell (9) and a joint ball (7) and which is adjustable between a state that releases the joint (3) and a state that locks the joint (3), wherein the bearing shell (9) is rigid and the joint ball (7) can be secured with a clamping action in the bearing shell (9) via a plurality of blocking elements (12), and wherein the bearing shell (9) has a two-part design consisting of an upper bearing shell (9a) and a lower bearing shell (9b), and a plurality of recesses (11) for the blocking elements (12) are formed in the lower bearing shell (9b),
**characterized in that**
the blocking elements (12), in the state that locks the joint (3), bear flat on the joint ball (7) and press the joint ball (7) with a clamping action into the bearing shell (9), and **in that** each blocking element (12) is pretensioned by a spring element (13) in the direction of bearing on the joint ball (7), and all the blocking elements (12) can be transferred, via a common actuation element (15), to a position releasing the joint (3).

2. Holding device according to Claim 1, **characterized in that** four recesses (11), distributed about the circumference of the bearing shell (9), are designed for in each case at least one blocking element (12) in the bearing shell (9).

3. Holding device according to Claim 1 or 2, **characterized in that** a clamping coating (14) is arranged on each blocking element (12) in the area where the blocking element (12) bears on the joint ball (7).

4. Holding device according to one of Claims 1 to 3, **characterized in that** each blocking element (12) has a conical shape, at least in the area where it bears on the joint ball (7).

5. Holding device according to one of Claims 1 to 4, **characterized in that** the common actuation element (15) is designed such that it pulls each blocking element (12) back from its bearing on the joint ball (7).

6. Holding device according to one of Claims 1 to 5, **characterized in that** the actuation element (15) can be actuated by application of an electrical voltage.

7. Holding device according to one of Claims 1 to 6, **characterized in that** the at least one actuation element (15) can be actuated via at least one piezoelectric actuator (16).

8. Holding device according to at least one of Claims 1 to 7, **characterized in that** the ball-and-socket joint (3) is sealed off from the environment by at least one sealing element (10) that bears on the joint ball (7).

## Revendications

1. Dispositif de retenue pour instruments médicaux, comprenant un bras de support (2) sur lequel au moins un instrument médical peut être fixé et comprenant au moins une articulation (3) pour positionner le bras de support (2) et/ou l'instrument médical, l'au moins une articulation (3) étant réalisée sous forme d'articulation à rotule (3) pourvue d'au moins une coque de palier (9) et d'une rotule d'articulation (7), l'articulation à rotule pouvant être déplacée entre un état libérant l'articulation (3) et un état bloquant l'articulation (3), la coque de palier (9) étant réalisée sous forme rigide et la rotule d'articulation (7) pouvant être fixée par le biais de plusieurs éléments de blocage (12) par serrage dans la coque de palier (9) et la coque de palier (9) étant réalisée en deux parties constituées d'une coque de palier supérieure (9a) et d'une coque de palier inférieure (9b) et plusieurs évidements (11) pour les éléments de blocage (12) étant réalisés dans la coque de palier inférieure (9b),
**caractérisé en ce que** les éléments de blocage (12), dans l'état bloquant l'articulation (3), s'appliquent à plat contre la rotule d'articulation (7) et pressent la rotule d'articulation (7) par serrage dans la coque de palier (9) et **en ce que** chaque élément de blocage (12) est précontraint par le biais d'un élément de ressort (13) dans la direction de l'application contre la rotule d'articulation (7) et tous les éléments de blocage (12) peuvent être transférés par le biais d'un élément d'actionnement commun (15) dans une position libérant l'articulation (3).

2. Dispositif de retenue selon la revendication 1, **caractérisé en ce que** quatre évidements (11) répartis sur la périphérie de la coque de palier (9) sont réalisés dans la coque de palier (9) pour à chaque fois au moins un élément de blocage (12).

3. Dispositif de retenue selon la revendication 1 ou 2, **caractérisé en ce qu'**une garniture de serrage (14) est disposée au niveau de chaque élément de blocage (12) dans la région de l'application de l'élément de blocage (12) contre la rotule articulation (7).

4. Dispositif de retenue selon l'une quelconque des revendications 1 à 3, **caractérisé en ce que** chaque élément de blocage (12) est réalisé sous forme conique au moins dans la région de l'application contre la rotule d'articulation (7).

5. Dispositif de retenue selon l'une quelconque des revendications 1 à 4, **caractérisé en ce que** l'élément d'actionnement commun (15) est réalisé de telle sorte qu'il tire en arrière chaque élément de blocage (12) hors de l'application contre la rotule articulation (7).

6. Dispositif de retenue selon l'une quelconque des revendications 1 à 5, **caractérisé en ce que** l'élément d'actionnement (15) peut être actionné par l'application d'une tension électrique.

7. Dispositif de retenue selon l'une quelconque des revendications 1 à 6, **caractérisé en ce que** l'au moins un élément d'actionnement (15) peut être actionné par le biais d'au moins un actionneur piézoélectrique (16).

8. Dispositif de retenue selon l'une quelconque des revendications 1 à 7, **caractérisé en ce que** l'articulation à rotule (3) est étanchéifiée vis-à-vis de l'environnement par le biais d'au moins un élément de joint d'étanchéité (10) s'appliquant contre la rotule d'articulation (7).
